(19) Europäisches Patentamt
European Patent Office
Office européen des brevets

(11) Publication number: 0 002 298
B1

(12) **EUROPEAN PATENT SPECIFICATION**

(45) Date of publication of patent specification: 21.01.81 Bulletin 81/03

(21) Application number: 78200317.2

(22) Date of filing: 25.11.78

(51) Int. Cl.³: C 07 C 126/02

# (54) Process and apparatus for the removal of ammonium carbamate from a urea-synthesis solution

(30) Priority: 30.11.77 NL 7713190

(43) Date of publication of application: 13.06.79 Bulletin 79/12

(45) Publication of the grant of the European patent: 21.01.81 Bulletin 81/03

(84) Designated Contracting States: BE DE FR GB SE

(56) References cited:
US - A - 3 356 723
US - A - 3 446 601
US - A - 3 936 500

(73) Proprietor: UNIE VAN KUNSTMESTFABRIEKEN B.V.
Postbus 45
NL - 3500 AA Utrecht/NL

(72) Inventor: Kaasenbrood, Petrus Johannes Cornelis
Irenelaan 9
NL - 6133 BE Sittard (NL)

(74) Representative: Roeffen, Wilhelmus Johannes Maria, et al
OCTROOIBUREAU DSM Postbus 9
NL - 6160 MA Geleen (NL)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 002 298 B1

Courier Press, Leamington Spa, England.

Process and apparatus for the removal of ammonium carbamate from a urea-synthesis solution

The invention relates to a process and apparatus for the removal of ammonium carbamate from a urea-synthesis solution containing urea, water, unconverted ammonium carbamate and $NH_3$ in which the urea-synthesis solution flows down along a wall as a thin film at a pressure of 11767.98 to 17651.97 KPa (120—180 atmospheres) and a temperature of 170°—200°C in counter-current flow to a gaseous stripping agent, while heat is applied, and stripped urea-synthesis solution and the gaseous mixture formed in stripping are discharged by way of collecting chambers.

A process of this type is known from United States Patent Specification 3,356,723. In the stripping treatment of a urea-synthesis solution described in this specification, this solution is contacted with gaseous $CO_2$ or $NH_3$ as stripping agent in counter-current flow with heating, preferably at the urea-synthesis pressure.

By adding gaseous $CO_2$ to the gaseous phase which is in equilibrium with the solution, the composition of the gaseous phase is changed so that it is no longer in equilibrium with the solution. As a result part of the ammonium carbamate contained in the liquid phase decomposes and the $NH_3$ and $CO_2$ then released pass over to the gaseous phase in order to restore the heterogeneous equilibrium. This desorption results in the equilibrium between ammonium carbamate, urea and water in the liquid phase being disturbed, so that part of the urea that has already formed hydrolyses into the starting products $NH_3$ and $CO_2$.

The rate of hydrolysis increases with temperature, but although the temperature at which the stripping treatment is effected in the known process is comparatively low and the retention time of the urea-synthesis solution in the stripping zone is kept short due to the use of heat exchange through the wetted wall, the hydrolysis of urea has some unfavourable influence, however small, on the conversion, and consequently on the efficiency of the process of preparing urea in which the stripping treatment is used.

Another secondary reaction that takes place during the stripping treatment is the formation of biuret, a compound that, when contained in urea fertilizer in too high a concentration, will cause damage to growing plants to which they are applied.

For this reason the biuret content of solid urea used for fertilizing purposes should not exceed 2.5% by weight. In urea fertilizer solutions that are applied to the leaves, the maximum allowable biuret content is only 0.3% by weight. The biuret content of the final product increases, as the temperature at which and the time during which a urea-synthesis solution is treated to decompose unconverted ammonium carbamate and remove $NH_3$, $CO_2$ and water from it increase.

In order to reduce both the hydrolysis of urea and the conversion of urea into biuret, it is consequently desirable to keep the time for which the urea-synthesis solution is exposed to high temperatures as short as possible.

In the stripping treatment described above, the temperature of the urea-synthesis solution rises owing to the application of heat. As a result, the equilibrium represented by the equation

$$NH_2COONH_4 \rightleftharpoons NH_2CONH_2 + H_2O$$

is shifted towards the right and urea is formed. If however ammonium carbamate is decomposed into $NH_3$ and $CO_2$, the said equilibrium is shifted towards the left and the urea hydrolyses. It has now been found that under said conditions as much urea hydrolyses as is formed after heating for at most 5 seconds. Heating for longer times consequently leads to reduction of the amount of urea in solution. When the stripping treatment is then continued with removal, instead of application, of heat, the decomposition of ammonium carbamate is continued, while the hydrolysis of urea is reduced to an acceptable level.

Thus the invention is directed to a process for the removal of ammonium carbamate from a urea-synthesis solution, in which the urea-synthesis solution flows down along a surface as a thin film at a pressure of from 11767.98 to 19613.30 KPa (120 to 200 atmospheres absolute) and a temperature of from 170 to 200°C in counter-current flow to a gaseous stripping agent, while heat is applied, and stripped urea-synthesis solution and the gaseous mixture formed in stripping are discharged to respective collecting chambers and discharged therefrom, characterized in that during the contact with the stripping agent the urea-synthesis solution is first heated to a temperature of from 180° to 195°C for not more than 5 seconds and then cooled to a temperature of from 155° to 175°C.

The process of the invention may expeditiously be carried out by effecting the stripping of the urea-synthesis solution on the internal walls of a plurality of heat-exchange tubes disposed substantially vertically in a tubular shell.

The upper part of such tubes along which the urea-synthesis solution flows down may be heated by means of a first liquid or gas and the lower part may be cooled by means of a second liquid or gas. Thus the said heating and cooling of the urea-synthesis solution in the said tubes is effected by heat-exchange with a heating agent contacting the upper part of the said tubes and with a cooling agent contacting the lower part of the said tubes.

However use is preferably made of a hot condensable gas, as it offers the possibility of varying the height of the unheated part of the wall by control of the condensate level. In such an embodiment the heating of the urea-synthesis solution in the said tubes is effected by heat-exchange with a hot condensable gaseous heating agent contacting the upper part of the said tubes and wherein control means are provided for determining the level at which discharge of the condensate of the said hot condensable gaseous heating agent from the space in the shell surrounding the tubes is effected. The said control means may be provided by at least one outlet for condensate at a lower level of said space. The level at which discharge of the condensate occurs may be controlled in a simple and flexible manner by selective operation of a plurality of outlets disposed at different levels along the shell. In this embodiment the cooling of the urea-synthesis solution is substantially effected by the endothermic decomposition of ammonium carbamate as a result of the contact with the stripping agent.

The cooling of the urea-synthesis solution being stripped is assisted by the contact with the stripping agent which is used at a temperature lower than that of the urea-synthesis solution, so that it also has a direct cooling effect. The best results are obtained when the temperature of the stripping agent is in the range 80—125°C. By the use of the process according to the invention, both the hydrolysis of urea and the conversion of urea into biuret are restricted. A further restriction can be achieved by keeping as short as possible the time between the moment at which the stripped urea-synthesis solution flows into the collecting chamber and the moment at which the pressure of the solution is lowered, and so a further drop in temperature takes place. This can be achieved by keeping the retention time in the collecting chamber for the stripped urea-synthesis solution less than 25 seconds. This retention time however should not be too short in view of the time of response of the level control in this collecting chamber, which aims at preventing gas from passing to the part of the installation beyond the expansion valve.

The invention also relates to an apparatus for carrying out the proposed process. The apparatus comprises a tubular shell containing a plurality of heat-exchanger tubes extending along the length thereof and entering a first collecting chamber at one end thereof for the collection of stripped urea-synthesis solution and entering a second collecting chamber at the other end thereof for the collection of gases, the said first collecting chamber being provided with discharge means for the discharge of stripped urea-synthesis solution therefrom and with inlet means for the introduction of gaseous stripping agent therein, the said second collecting means being provided with discharge means for the discharge of gases therefrom, the said tubes each being provided with inlet means for the introduction therein of urea-synthesis solution to be stripped at a location adjacent to but not in the said second collection chamber whereby urea-synthesis solution may flow down the inner wall of each of the said tubes as a thin film when the said tubes are substantially in a vertical disposition, and the said tubular shell is provided with an inlet means adjacent to but not in the said second collection chamber for the introduction into the tubular shell of a hot condensable heating agent, and with outlet means adjacent to but not in the said first collecting chamber for the removal from the tubular shell of condensed heating agent; characterized in that the said outlet means for condensed heating agent comprises a plurality of discharge valves spaced along the length of the tubular shell wall, being independently operable.

The invention is hereinafter further described and illustrated in the accompanying drawing, which is a diagrammatic vertical section of one embodiment of a stripping apparatus that is suitable for carrying out the process according to the invention. The urea-synthesis solution to be treated, which is at a temperature of for example between 180° and 185°C, is fed to the stripping apparatus through inlet 1 leading to liquid-distributing space 2 between the upper tube plate 3 and an upper wall 4 which separates this space from gas-collecting chamber 5. The pressure at which the solution is treated may be the urea-synthesis pressure, e.g. 13729.31 KPa (140 atmospheres absolute). If higher synthesis pressures are used, the urea-synthesis solution may first be expanded if desired, before it is fed to the stripping apparatus. From the liquid-distributing space, the solution is evenly distributed over the vertical tubes 6 that are fitted in the upper tube plate 3 and the lower tube plate 7, for instance by maintaining a sufficiently high liquid level in said space. Many hundreds of these tubes may be provided, but the drawing shows diagrammatic sections of only two and the centre lines of some other tubes. The head of each tube is equipped with a liquid distributor which distributes the urea-synthesis solution over the inner wall of the tube so that it flows down as a thin film inside the tubes. For the sake of simplicity these liquid distributors are indicated merely by the openings 8. The preferred tube length is 6—8 m and the internal diameter is preferably 20—30 mm. The liquid load per tube is 80—90 kg/hour to avoid flooding. The solution flows down along the inner wall of the tubes 6 with a speed of about 1 m/s in counter-current flow to a stripping gas, which may be for instance $CO_2$, $NH_3$ or an inert gas or a mixture of two or more of these gases. This stripping gas, which has a temperature of e.g. 120°C, flows from distributor 10 with feed pipe 11 through the liquid-collecting chamber 9 into the tubes.

The heat required for the decomposition of ammonium carbamate in the urea-synthesis solution is obtained by heating the tubes 6 by means of a gaseous or liquid heating agent that is introduced through inlet 12 into the top of the shell of the stripping apparatus. To effect proper heat transfer, partitions 13 have been mounted in the tubular shell. The stripping apparatus shown here is equipped for the use of saturated steam as a heating agent. This steam, which has a temperature of for example 210—220°C, condenses in the tubular shell of the stripping apparatus. The resulting condensate is discharged through one of the outlets 14, 15, or 16 fitted with valves and at various heights to the lower part of the tubular shell. By appropriate operation of the valves the level to which the condensate surrounds the tubes is controlled. The urea-synthesis solution flowing down in tubes 6 is now heated from the inlet openings 8 to the parts of the tubes surrounded by condensate. This permits the temperature to rise to about 195°C. When the solution flows through this lower part of the tubes, no more heat is applied, but as a result of the contact with the comparatively cold stripping gas and owing to its stripping action, heat is given off or consumed by the decomposition of ammonium carbamate and evaporation of the $NH_3$ and $CO_2$ then released. If so desired, the temperature of the condensate may be lowered by feeding colder water. By appropriate operation of the outlets 14, 15 and 16 the level at which the condensate is drained from the tubular shell is so chosen that it is reached within 5 seconds by the liquid flowing down in the tubes.

In principle, it is also possible to heat and cool with separate gases or liquids. If so, the space surrounding the stripping tubes in the shell may be fitted with a horizontal partition that fully separates the heating zone from the cooling zone. Here too saturated steam may be used as the heating agent, while the cooling agent may be, e.g. process condensate or boiler feed water that have to be heated, so that the heat discharged is utilized directly. In this embodiment however, it is less simple to change the period of heating the urea-synthesis solution by variation of the height of the heating zone than in the embodiment described above.

The stripped urea-synthesis solution flowing from tubes 6 and having a temperature of, e.g., 160°C is collected in liquid-collecting chamber 9, from where it is discharged as soon as possible, e.g. within 20 seconds, through outlet 17. However, a minimum liquid level should be maintained in order to prevent that part of the stripping gas supplied through line 11 and distributor 10 is discharged along with the stripped urea-synthesis solution. A very low and still effective liquid level, and hence a very short retention time of the liquid in collecting chamber 9, can be obtained by fitting a so-called vortex breaker right over outlet 17. To reduce the harmful effect of the retention in liquid-collecting chamber 9, one or more heat-exchanging members may be fitted in this chamber by means of which the stripped urea-synthesis solution can be cooled. The $NH_3$ and $CO_2$ expelled from the urea-synthesis solution by the stripping treatment and a small equilibrium amount of water vapour are passed, together with the stripping agent used, from the top of tubes 6 through opening 18 to a gas-collecting chamber 5, from where they are returned through outlet 19 for example to a condenser or the urea reactor.

## Claims

1. A process for the removal of ammonium carbamate from a urea-synthesis solution, in which the urea-synthesis solution flows down along a surface as a thin film at a pressure of from 11767.98 to 19613.30 KPa (120 to 200 atmospheres absolute) and a temperature of from 170 to 200°C in counter-current flow to a gaseous stripping agent, while heat is applied, and stripped urea-synthesis solution and the gaseous mixture formed in stripping are discharged to respective collecting chambers and discharged therefrom, characterized in that during the contact with the stripping agent the urea-synthesis solution is first heated to a temperature of from 180° to 195°C for not more than 5 seconds and then cooled to a temperature of from 155° to 175°C.

2. A process according to Claim 1, wherein the stripping of the said urea-synthesis solution is effected on the internal surfaces of a plurality of heat-exchange tubes disposed substantially vertically in a tubular shell.

3. A process according to Claim 2, wherein the said heating and cooling of the urea-synthesis solution in the said tubes is effected by heat-exchange with a heating agent contacting the upper part of the said tubes and with a cooling agent contacting the lower part of the said tubes.

4. A process according to Claim 2, wherein the said heating of the urea-synthesis solution in the said tubes is effected by heat-exchange with a hot condensable gaseous heating agent contacting the upper part of the said tubes and wherein control means are provided for determining the level at which the condensate of the said hot condensable gaseous heating agent from the space in said shell surrounding the tubes is effected.

5. A process according to Claim 3, wherein the said control means is provided by at least one outlet for condensate at a lower level thereof.

6. A process according to Claim 5, wherein a plurality of said outlets is provided at different levels, the level at which discharge of the condensate occurs being determined by selective operation of said outlets.

7. A process according to any of Claims 2 to 6,

wherein cooling of the heated urea-synthesis solution in said tubes is effected by using a gaseous stripping agent at a temperature lower than the temperature of the urea-synthesis solution at the end of the heating period thereof.

8. A process according to Claim 7, wherein the temperature of the said gaseous stripping agent is in the range of from 80° to 125°C.

9. A process according to any of Claims 1 to 8, wherein the said gaseous stripping agent is $CO_2$.

10. A process according to any of Claims 1 to 9, wherein the stripped urea solution after entry into the collecting chamber therefor is retained therein for less than 25 seconds before discharge therefrom.

11. A process according to Claim 10, wherein the stripped urea solution is cooled when in the said collecting chamber.

12. A urea solution obtained by a process according to any of Claims 1 to 11.

13. Apparatus for the removal of ammonium carbamate from a urea-synthesis solution, comprising a tubular shell containing a plurality of heat-exchanger tubes extending along the length thereof and entering a first collecting chamber at one end thereof for the collection of stripped urea-synthesis solution and entering a second collecting chamber at the other end thereof for the collection of gases, the said first collecting chamber being provided with discharge means for the discharge of stripped urea-synthesis solution therefrom and with inlet means for the introduction of gaseous stripping agent therein, the said second collecting means being provided with discharge means for the discharge of gases therefrom, the said tubes each being provided with inlet means for the introduction therein of urea-synthesis solution to be stripped at a location adjacent to but not in the said second collection chamber whereby urea-synthesis solution may flow down the inner wall of each of the said tubes as a thin film when the said tubes are substantially in a vertical disposition, and the said tubular shell is provided with an inlet means adjacent to but not in the said second collection chamber for the introduction into the tubular shell of a hot condensable heating agent, and with outlet means adjacent to but not in the said first collecting chamber for the removal from the tubular shell of condensed heating agent; characterized in that the said outlet means for condensed heating agent comprises a plurality of discharge valves spaced along the length of the tubular shell wall, being independently operable.

**Revendications**

1. Procédé pour éliminer le carbamate d'ammonium à partir d'une solution de synthèse d'urée, en faisant descendre la solution de synthèse d'urée, à une pression de 11767,98—19613,30 kPa et à une température de 170—200°C, en contre-courant avec un agent de strippage gazeux, sous adduction de chaleur et en évacuant la solution de synthèse d'urée strippée et la mélange gazeux formé lors du strippage à des espaces collecteurs respectifs d'où on les évacue, caractérisé en ce que la solution d'urée de synthèse est d'abord chauffée pendant tout au plus 5 sec., lors du contact avec l'agent de strippage, jusqu'à une température de 180—195°C et puis refroidie jusqu'à une température de 155—175°C.

2. Procédé selon la revendication 1, caractérisé en ce qu'on effectue le strippage de la solution de synthèse d'urée sur les parois intérieures d'un certain nombre de tubes-échangeurs de chaleur qui sont placés essentiellement verticaux dans une chemise tubulaire.

3. Procédé selon la revendication 2, caractérisé en ce qu'on réalise le chauffage et le refroidissement de la solution de synthèse d'urée dans les tubes moyennant échange de chaleur avec un agent de chauffage, qui se met en contact avec la partie supérieure des tubes et avec un agent de refroidissement, qui se met en contact avec la partie inférieure des tubes.

4. Procédé selon la revendication 2, caractérisé en ce qu'on réalise le chauffage de la solution de synthèse d'urée dans les tubes moyennant un agent de chauffage gazeux chaud et condensable qui se met en contact avec la partie supérieure des tubes alors qu'on a prévu des organes de réglage servant à régler le niveau auquel le condensat de l'agent de chauffage gazeux chaud et condensable est évacué depuis la chemise tubulaire entourant les tubes.

5. Procédé selon la revendication 3, caractérisé en ce que les organes de réglage sont munis d'au moins d'un orifice d'évacuation pour le condensat, lequel orifice est situé à un niveau plus bas.

6. Procédé selon la revendication 5, caractérisé en ce que plusieurs orifices d'évacuation sont aménagés à différents niveaux et que le niveau auquel se fait l'évacuation du condensat est déterminé par la commande sélective des orifices d'évacuation.

7. Procédé selon l'une des revendications 1—6, caractérisé en ce que le refroidissement de la solution synthèse d'urée chauffée se fait dans les tubes en utilisant un agent de strippage gazeux ayant une température inférieure à celle de la solution d'urée de synthèse à la fin de la période de chauffage de celle-ci.

8. Procédé selon la revendication 7, caractérisé en ce que la température de l'agent de strippage gazeux se situe entre 80 et 125°C.

9. Procédé selon l'une des revendications 1 à 8, caractérisé en ce que l'agent de strippage gazeux est du $CO_2$.

10. Procédé selon l'une des revendications 1—9, caractérisé en ce que la solution d'urée

strippée, après son introduction dans l'espace collecteur, y séjourne moins de 25 sec. avant de sortir de cet espace.

11. Procédé selon la revendication 10, caractérisé en ce que la solution d'urée strippée est refroidie dans l'espace collecteur.

12. Solution d'urée obtenue par la mise en oeuvre du procédé selon l'une ou l'autre des revendications 1—11.

13. Dispositif pour éliminer le carbamate d'ammonium à partir d'une solution de synthèse d'urée, composé d'une chemise tubulaire contenant un certain nombre de tubes échangeurs de chaleur faisant saillie de l'espace dans la chemise tubulaire, dont un bout se décharge dans un premier espace collecteur destiné à recueillir la solution d'urée de synthèse strippée et l'autre bout se décharge dans un deuxième espace collecteur destiné à recueillir des gaz, alors que le premier espace collecteur est muni d'organes pour évacuer la solution de synthèse d'urée strippée et d'organes pour amener l'agent de strippage gazeux à cet espace, que le deuxième espace collecteur est muni d'organes pour en évacuer du gaz, que les tubes sont munis chacun d'organes pour y amener la solution de synthèse d'urée strippée à un endroit annexe au deuxième espace collecteur mais ne se déchargeant pas là-dedans, que la solution de synthèse d'urée peut descendre sous forme d'un mince film le long de la paroi intérieure de chacun de ces tubes quand ceux-ci sont montés essentiellement verticaux, que la chemise tubulaire est munie de moyens, annexes au deuxième espace collecteur mais ne se déchargeant pas là-dedans, destinés à introduire dans la chemise tubulaire un agent de chauffage chaud condensable, ainsi que de moyens, annexes au premier espace collecteur mais ne se déchargeant pas là-dedans, pour évacuer l'agent de chauffage condensé de la chemise tubulaire, caractérisé en ce que les moyens d'évacuation pour l'agent de chauffage condensé sont munis d'un certain nombre de soupapes d'arrêt placées sur la longueur de la paroi de la chemise tubulaire et pouvant être commandées l'une indépendamment de l'autre.

## Patentansprüche

1. Verfahren zum Entfernen von Ammoniumcarbamat aus einer Harnstoffsyntheselösung, wobei man die Harnstoffsyntheselösung bei einem Druck von 11767,98 bis 19613,30 kPa und einer Temperatur von 170—200°C als ein dünner Film im Gegenstrom mit einem gasförmigen Strippmittel an einer Oberfläche entlang nachströmen lässt, während Wärme zugeführt wird und gestrippte Harnstoffsyntheselösung sowie das während des Strippens anfallende gasförmige Gemisch in Sammelräume eingeführt und aus ihnen abgeführt werden, dadurch gekennzeichnet, dass man die Harnstoffsyntheselösung während der Berührung mit dem Strippmittel erst höchstens 5 Sek. lang auf eine Temperatur von 180—195°C erhitzt und danach auf eine Temperatur von 155—175°C abkühlt.

2. Verfahren gemäss Anspruch 1, dadurch gekennzeichnet, dass man das Strippen der Harnstoffsyntheselösung an den Innenwänden von in einem rohrförmigen Mantel im wesentlichen senkrecht aufgestellten Wärmeaustauschrohren stattfinden lässt.

3. Verfahren gemäss Anspruch 2, dadurch gekennzeichnet, dass man das Erhitzen und das Abkühlen der Harnstoffsyntheselösung in den Rohren durch Wärmeaustauschung mit einem Heizmittel bewerkstelligt, das mit dem oberen Teil der Rohre in Berührung gerät und mit einem Kühlmittel, das mit dem unteren Teil der Rohre in Berührung gerät.

4. Verfahren gemäss Anspruch 2, dadurch gekennzeichnet, dass man das Erhitzen der Harnstoffsyntheselösung in den Rohren mit einem warmen, kondensierbaren, gasförmigen Heizemittel bewerkstelligt, das mit dem oberen Teil der Rohre in Berührung gerät und dass Mittel zur Regelung des Niveaus, auf dem die Abfuhr des Kondensats des warmen, kondensierbaren, gasförmigen Heizmittels aus der den Raum um die Rohre umgebenden Wand stattfindet, vorgesehen sind.

5. Verfahren gemäss Anspruch 3, dadurch gekennzeichnet, dass die Mittel zur Regelung des Niveaus von mindestens einer Abfuhr für Kondensat auf ein niedrigeres Niveau gebildet werden.

6. Verfahren gemäss Anspruch 5, dadurch gekennzeichnet, dass mehrere Abfuhren auf verschiedenen Niveaus angebracht sind und dass das Niveau, auf dem die Kondensatabfuhr stattfindet, durch selektive Bedienung der Abfuhren eingestellt wird.

7. Verfahren gemäss einem der Ansprüche 1—6, dadurch gekennzeichnet, dass die Abkühlung der erhitzten Harnstoffsyntheselösung in den Rohren stattfindet, indem ein gasförmiges Strippmittel mit einer Temperatur unter der der Harnstoffsyntheselösung am Ende ihrer Heizperiode verwendet wird.

8. Verfahren gemäss Anspruch 7, dadurch gekennzeichnet, dass die Temperatur des gasförmigen Strippmittels zwischen 80 und 125°C liegt.

9 Verfahren gemäss einem der Ansprüche 1—8, dadurch gekennzeichnet, dass das gasförmige Strippmittel $CO_2$ ist.

10. Verfahren gemäss einem der Ansprüche 1—9, dadurch gekennzeichnet, dass die gestrippte Harnstofflösung, nach Eintreten in die Sammelkammer, in ihr weniger als 25 Sek. verweilt, ehe sie die Kammer verlässt.

11. Verfahren gemäss Anspruch 10, dadurch gekennzeichnet, dass die gestrippte Harnstofflösung in der Sammelkammer abgekühlt wird.

12. Harnstofflösung hergestellt nach einem Verfahren gemäss einem der Ansprüche 1—11.

13. Vorrichtung zum Entfernen von Ammoniumcarbamat aus einer Harnstoffsyntheselösung, bestehend aus einem Mantelraum, der sich über seiner Länge erstreckende Wärmeaustauschrohre enthält, die auf einer Seite in eine erste Kammer zum Sammeln gestrippter Harnstoffsyntheselösung und am anderen Ende in eine zweite Kammer zum Sammeln von Gasen ausmünden, wobei die erste Sammelkammer mit Mitteln zum Abführen gestrippter Harnstoffsyntheselösung und solcher zum Zuführen des gasförmigen Strippmittels, die zweite Kammer mit Mitteln zum Abführen von Gas und die Rohre je mit Mitteln zum Zuführen der zu strippenden Harnstoffsyntheselösung versehen sind, an einer Stelle, grenzend an, aber nicht in der zweiten Sammelkammer, wodurch Harnstoffsyntheselösung als ein dünner Film an der Innenwand eines jeden Rohrs nach unten strömen kann, wenn die Rohre im wesentlichen senkrecht aufgestellt sind, und wobei der Mantelraum mit Mitteln grenzend an, jedoch nicht in der zweiten Sammelkammer zum Zuführen in den rohrförmigen Mantel eines warmen kondensierbaren Heizmittels und mit Mitteln grenzend an, jedoch nicht in der ersten Sammelkammer zum Abführen aus dem Mantelraum von kondensiertem Heizmittel versehen sind, dadurch gekennzeichnet, dass die Abfuhrmittel für kondensiertes Heizmittel über die Länge der Wand des Mantelraums angeordnete Abfuhrventile sind, die unabhängig von einander gesteuert werden können.

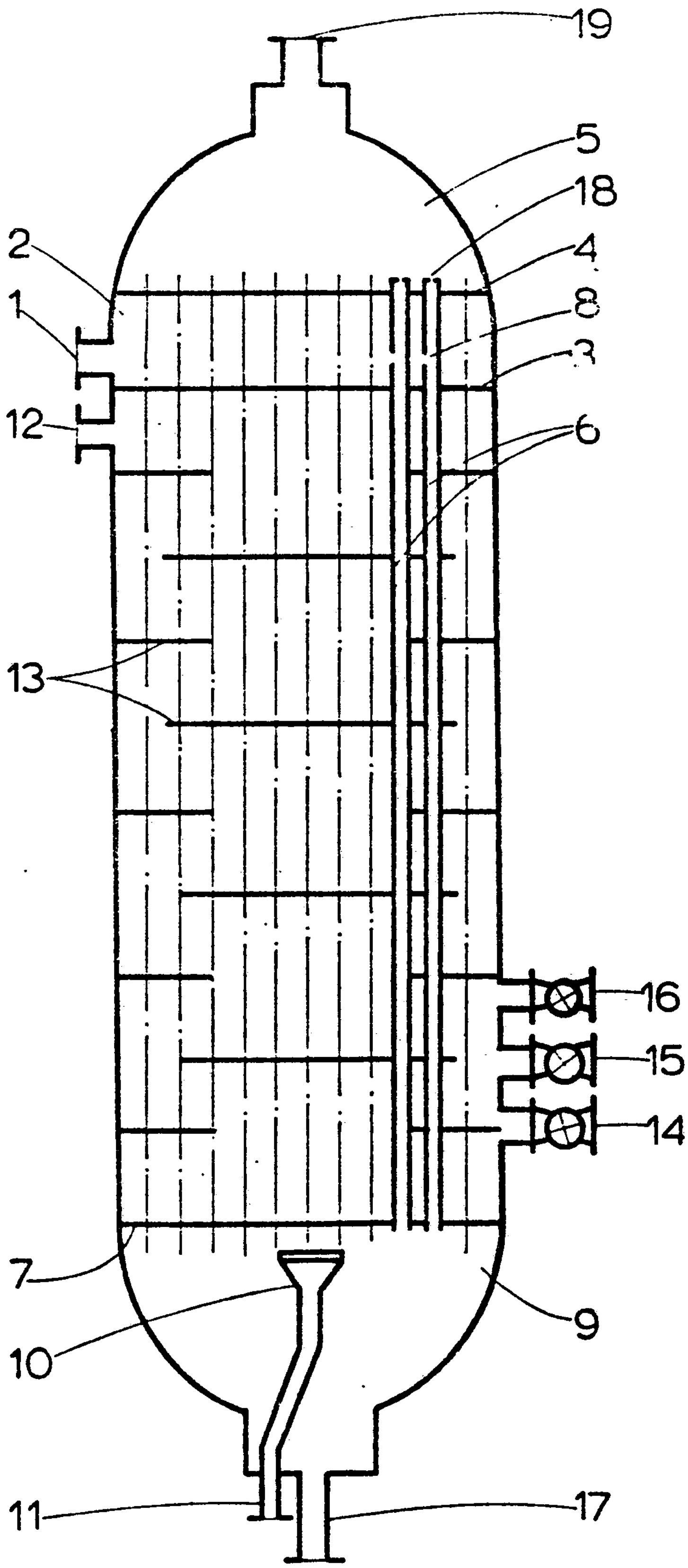
19
5
18
2
4
1
8
3
12
6
13
16
15
14
7
9
10
11
17